(19) Europäisches Patentamt
European Patent Office
Office européen des brevets

(11) Publication number: **0 168 175**
**B1**

(12) # EUROPEAN PATENT SPECIFICATION

(45) Date of publication of patent specification: **19.11.87**

(51) Int. Cl.$^4$: **C 07 C 93/06**

(21) Application number: **85304129.1**

(22) Date of filing: **11.06.85**

(54) **Preparation of tamoxifen.**

(30) Priority: **12.06.84 GB 8414902**
**14.12.84 GB 8431600**

(43) Date of publication of application:
**15.01.86 Bulletin 86/03**

(45) Publication of the grant of the patent:
**19.11.87 Bulletin 87/47**

(84) Designated Contracting States:
**CH DE FR LI**

(56) References cited:
**EP-A-0 126 470**
**GB-A-1 146 405**

(73) Proprietor: **NATIONAL RESEARCH DEVELOPMENT CORPORATION**
**101 Newington Causeway**
**London SE1 6BU (GB)**

(72) Inventor: **Coe, Paul Leslie**
**17 Meadowbrook Road**
**Northfield Birmingham B31 1NE (GB)**
Inventor: **Scriven, Clare Elizabeth**
**Tythe Barn House The Drive Chestfield**
**Whitstable Kent CT5 3NS (GB)**

(74) Representative: **Percy, Richard Keith**
**Patent Department National Research Development Corporation**
**101 Newington Causeway**
**London SE1 6BU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 168 175 B1

**Description**

BACKGROUND OF THE INVENTION

1. *Field of the invention*

This invention relates to the preparation of Tamoxifen.

DESCRIPTION OF THE PRIOR ART

Tamoxifen is Z(1,2-diphenyl)-1-[4-(2-N,N-dimethylaminoethoxy)phenyl]-1-butene of formula

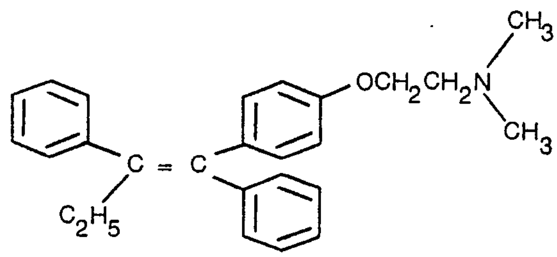

(I)

Its anti-oestrogenic properties have led since 1971 to its clinical use in the treatment of malignant tumours, especially oestrogen receptor-positive breast cancer. The Z isomer only is useful for this application and its purity, with respect to absence of the E isomer as contaminant is critical, since the latter is an oestrogen agonist and may counteract the anti-oestrogenic effect of the Z isomer. In this specification the Z isomer is referred to simply as Tamoxifen, the E isomer is referred to as E Tamoxifen, and mixtures of the isomers as EZ Tamoxifen.

GB—A—1064629 (I.C.I.) describes separating EZ Tamoxifen into the individual isomers by crystallization of the EZ mixture or a salt (generally the citrate) from a suitable solvent. A separation of EZ Tamoxifen into its isomers by crystallisation from methanol and petroleum ether respectively has also been described by the I.C.I. investigators G. R. Bedford and D. N. Richardson, Nature *212,* 733 (1966) with the semi-dimethylamide of dibenzoyl D-tartaric acid. The method depended on the selective separation of this salt of the Z isomer in a butyl acetate medium.

An intermediate in the synthesis of Tamoxifen is the phenol 1,2-diphenyl-1-(4-hydroxyphenyl)-1-butene, of formula

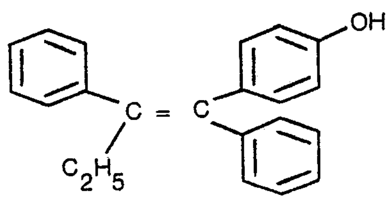

(4)

The conversion of EZ mixtures of this phenol into EZ Tamoxifen by reaction with 2-dimethylaminoethyl chloride is known, see Example 2 of GB—A—1,013,907. Unfortunately, no satisfactory procedure has been described for separating the EZ isomers of the phenol of formula (4) and therefore the preparation of this intermediate does not help to provide a better route to Tamoxifen.

European Patent Application 84105719.3 (Bristol-Myers Co.) filed 18 May 1984 and published 28 November 1984 as No. 126470A describes a preparation of EZ Tamoxifen in which the Z:E isomer ratio in the crude reaction product is about 2:1. The Bristol-Myers process makes use of the McMurry reaction for the preparation of olefins from ketones.

The McMurry reaction has been known since 1974 and has recently been reviewed by its discoverer, Dr. J. E. McMurry, in Accounts of Chemical Research *16,* 405—411 (1983). The reaction involves a simultaneous reduction and coupling of two ketone or aldehyde functions to produce a molecule in which the carbon atoms of the two carbonyl groups are coupled together to form an olefinic double bond. The reduction requires a titanium compound such as the trichloride and a powerful reducing agent such as lithium or potassium. The reaction has mainly been used to make dimers by the reaction of two molecules of the same ketone or aldehyde or for cyclisation of an aliphatic omega-diketone or dialdehyde to produce macrocyclic compounds by coupling the chain-end carbonyl functions. There have been a few experiments in coupling together different carbonyl compounds, thus forming unsymmetrical olefins, see J. E. McMurry and L. R. Krespski, Journal of Organic Chemistry, *41,* 3929—3930 (1976). Benzophenone or fluorenone was reacted with acetone, cyclohexanone, 3-cholestanone, hexanal, di-t-butylketone, cycloheptanone or acetophenone. In all cases but one, the mixed product predominated over the self-coupled product. The authors postulated that whereas self-coupling proceeds by a free-radical mechanism, mixed coupling has a different mechanism. Dianions are produced by one ketone, which attaches to the other by nucleophilic

addition. The authors suggest that good yields of mixed-coupled product of any two ketones should be obtained if benzophenone or fluorenone is reduced to a dianion before the other is reduced to an anion-radical. However, the mixed-coupling McMurry reaction to yield olefines has not been pursued to any great extent and in the 1983 review, *supra*, Dr. McMurry concluded that this version of his reaction was not of general synthetic use.

In the Bristol-Myers process propiophenone is coupled with 4-(2-N,N-dimethylaminoethoxy)benzophenone, according to the reaction scheme:—

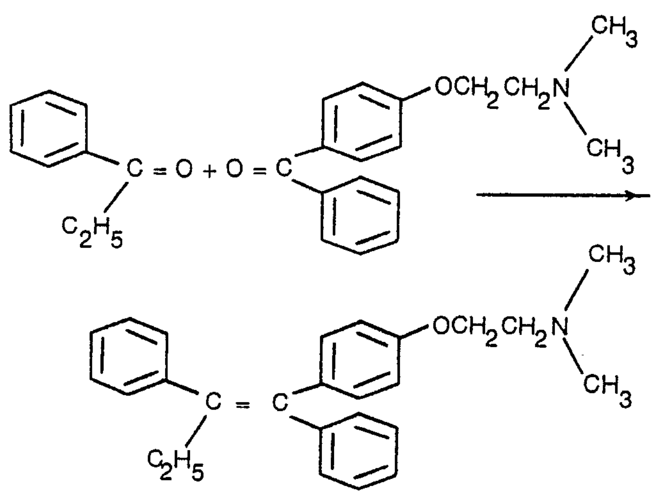

Surprisingly the mixed coupling reaction gives a product in which the desired Z-isomer predominates. Working up the reaction product gave a reported yield of 51.2% of a product said to be greater than 85% pure EZ Tamoxifen in which the Z:E isomer ratio was 97.5:1. The Bristol-Myers Co. specification does not describe the preparation of 4-(2-N,N-dimethylaminoethoxy)benzophenone, but the present inventors have obtained this compound from 4-hydroxybenzophenone in a yield of 79.3%. The *maximum* overall yield of pure product obtainable the Bristol-Myers process is therefore 79.3 × 51.2 × 85% = 34.5%. If the EZTamoxifen mixture had been 90% pure the maximum yield would have been 36.5%. Such yield figures are, however, optimistic, considering that purification of the EZ Tamoxifen mixture would inevitably result in a greater than theoretical loss of yield, so that a yield of pure product of, say, 30% would be a more realistic expectation.

SUMMARY OF THE INVENTION

At the first priority date (12 June 1984) of the present application the inventors were unaware of the earlier pending patent application of Bristol-Myers Co. and had independently made the surprising finding that when propiophenone is coupled with an appropriately substituted benzophenone, the mixed coupling reaction gives a product in which the Z isomer predominates very substantially over the E. As far as was known to the inventors, no one had previously investigated the mixed-coupling McMurry reaction for stereospecific synthesis.

The present inventors' research, however, was not limited to the one-step reaction of propiophenone with 4-(2-N,N-dimethylaminoethoxy)benzophenone. They have found that the yield of Tamoxifen can be increased by proceeding in two steps, in which the McMurry reaction is carried out on propiophenone with a 4-(2-haloethoxy)benzophenone to yield an intermediate, 1,2-diphenyl-1-[4-(2-haloethoxy)phenyl]-1-butene, which is then reacted with dimethylamine. To compare the yield with the Bristol-Myers process one has to consider 4-hydroxybenzophenone as the starting point. The present inventors have obtained an overall yield of 87.5 × 54.2 × 83.0% = 39.4% of a product of analytical purity. However, the Bristol-Myers product was not of analytical purity (apart from the 15% or so impurities, it had an E isomer content of 1%). Probably a fair comparison would be between the 34.5% yield from the Bristol-Myers process and the 87.5 × 54.2 × 94.7% = 44.9% yield of Tamoxifen obtained by the present process before the final chromatography required to give an analytically pure product. At all events it is clear that the present process can give a significant yield advantage. It is surprising that the present process, although superficially a less direct route, involving an extra step, gives a yield advantage and this could not have been foreseen. Accordingly the present invention is believed importantly distinct from and unobvious over the Bristol-Myers process.

According to the present invention there is provided a process of preparing Tamoxifen which comprises:

(1) reacting propiophenone with a 4-(2-haloethoxy)benzophenone of formula

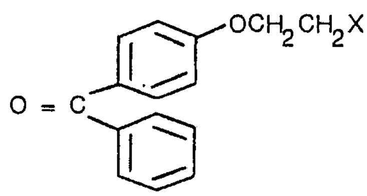

(2)

X being chlorine, bromine or iodine, in a substantially dry and inert atmosphere and in a medium containing a reducible titanium compound and a reducing agent effective to generate titanium of substantially zero valence state, to give the intermediate compound 1,2-diphenyl-1-[4-(2-haloethoxy)-phenyl]-1-butene, of formula

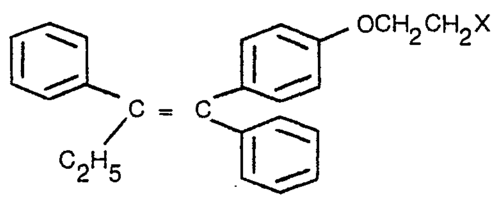

(3)

X being as defined above, and

(2) reacting the intermediate compound of formula (3) with dimethylamine.

DESCRIPTION OF THE PREFERRED EMBODIMENTS

The 4-(2-haloethoxy)benzophenone reactant can be prepared from 4-hydroxybenzophenone, and a 1,2-dihaloethane. Preferably the reaction is carried out under phase transfer conditions, with the aid of a quaternary ammonium salt phase transfer catalyst, which can be any of ones well known *per se.* Alternatively a strong proton extracting agent such as a sodium alkoxide is used in the reaction.

McMurry olefin synthesis reaction conditions require a reducible titanium compound and a reducing agent effective to generate titanium of low valence state, believed to be substantially zero, e.g. not normally greater than 1.2 per average atom. It is believed that the titanium is reduced to a valence state of either 2 or 0 and that typically, to yield olefins, 0 to 60% of the atoms are 2-valent and the balance zero-valent. The preferred titanium salt is a chloride, but any salt of tri- or tetravalent titanium is normally usable. The tetrachloride is currently preferred to the trichloride. Reducing agents include lithium, lithium aluminium hydride, potassium, zinc or a zinc-copper couple. (A zinc-copper couple can be prepared from zinc dust and a small amount of a copper salt in deoxygenated water). Preferably the reducible titanium compound is the trichloride and the reducing agent is lithium, or the titanium compound is the tetrachloride and the reducing agent is zinc. It is particularly advantageous to heat $TiCl_4$ with zinc, preferably at a temperature of 50 to 100°C, to pre-prepare the reagent, before adding it to the ketones.

The McMurry olefin synthesis reaction used in the invention is conveniently carried out using an excess of the reducing agent relative to the titanium salt of e.g. from 2:1 to 5:1 molar.

The reaction is carried out in a substantially inert atmosphere, e.g. in argon or nitrogen, to prevent premature oxidation of the reducing agent. The atmosphere should also be substantially free of moisture.

The reaction can be carried out at room temperature, but usually requires heat (above 25°C) to proceed faster. A temperature range of 15 to 100°C, preferably 15 to 70°C, and most preferably 40 to 70°C, is therefore usual for this stage.

The reaction is carried out in an inert liquid medium, which is normally a solvent for the ketone reactants, preferably 1,2-dimethoxyethane or tetrahydrofuran.

The titanium compound will ordinarily be present in a large molar excess over the two ketones. The reducing agent is preferably used in a molar ratio of at least 3:1 relative to the titanium compound. Preferably ketones are used in substantially equimolar proportions. Otherwise the ketone in excess will usually self-couple, producing unwanted side products.

After the McMurry reaction has been used to produce a compound having a 2-chloroethoxy, 2-bromoethoxy, or 2-iodoethoxy substituent in the 4-position of the relevant benzene ring, the product is readily convertible into Tamoxifen or a Z-enriched EZ Tamoxifen mixture in one step, using dimethylamine. This step is normally carried out in a closed environment, such as a sealed vessel, and at a temperature of from 60 to 100°C, preferably 70 to 90°C.

Considerable variation is possible in work-up procedures, which can comprise crystallisation, absorption on solids and chromatography. It is desirable to remove any dark or coloured material produced from the McMurry reaction and this will ordinarily require an absorption or chromatography step. On the other hand, work-up from the product of the dimethylamine reaction will ordinarily be minimal.

The following Examples illustrate the invention. Temperatures are in °C.
NMR spectral data refer to the following assignations of letters to H- atoms:—

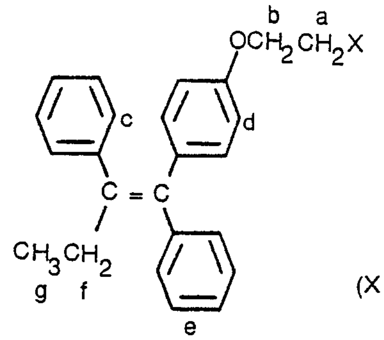

(X = Cl, Br or I)

and *mutatis mutandis* for the E isomers.

Example 1

Preparation of Tamoxifen (Z 1-[4-(2-dimethylaminoethoxy)phenyl]-1,2-diphenylbut-1-ene)

(a) *Preparation of 4-(2-chloroethoxy)benzophenone*

4-Hydroxybenzophenone (13.23 g, 70 mmol) was added to a solution of sodium (3.2 g) in ethanol (60 ml) and the resulting deep red solution stirred for 1 hour at room temperature. The solvent was removed by rotary evaporation to leave the organic sodium salt. Toluene (50 ml) and 1-bromo-2-chloroethane (20 g, 140 mmol) were added and the mixture stirred at reflux for 18 hours. After cooling to room temperature the solid material was removed by filtration and the solvent in the filtrate was evaporated to give a pale brown oil. Crystallisation from ethanol/water gave 4-(2-chloroethoxy)-benzophenone as pale cream flakes (12.1 g, 73%), m.p. 78°.

(b) *Preparation of 1-[4-(2-chloroethoxy)phenyl]-1,2-diphenylbut-1-ene*

Lithium pieces (0.45 g, 65 mmol) were added to a slurry of titanium trichloride (2.87 g, 18.6 mmol) in dry dimethoxyethane (30 ml) under argon and stirred under reflux for 1 hour. After allowing the black mixture to cool to room temperature, a solution of 4-(2-chloroethoxy)benzophenone (0.54 g, 2.0 mmol) and propiophenone (0.31 g, 2.3 mmol) in dry dimethoxyethane (20 ml) was added and the whole stirred at room temperature for 2 hours. The mixture was then refluxed with stirring for 20 hours.

After allowing the black suspension to cool to room temperature, petroleum ether (b.p. 60—80°, 50 ml) was added and the mixture was stirred for 15 minutes. The organic layer was decanted off and the solvent was evaporated to leave a brown oil (0.77 g).

The oil was purified by passage down a silica gel column, eluting with hexane/ethyl acetate 9:1 v/v. Fractions were collected and monitored by TLC and the fractions having Rf = 0.44 combined, the solvent was evaporated to give a colourless oil (0.51 g, 68%). From NMR, it was estimated that the Z:E isomer ratio was between 6:1 and 9:1. This oil was dissolved in isopropanol (20 ml) and the white crystals which separated on standing were removed by filtration, washed with isopropanol, and dried in a desiccator (0.29 g, 34%), m.p. 63—65°. NMR showed these to be pure Z 1-[4-(2-chloroethoxy)-phenyl]-1,2-diphenylbut-1-ene.

Elemental analysis. Found C 79.7, H 6.3; $C_{24}H_{23}ClO$ requires C 79.4, H 6.4%. NMR, 60 mHz on EZ mixture:

| | E | Z | |
|---|---|---|---|
| a and b | 3.53 | 4.34 (m) | 4H |
| c or e | 7.07 (s) | 7.07 (s) | 5H |
| d | 6.53—7.05 | 6.34—6.85 | 4H |
| e or c | 7.21 (s) | 7.21 (s) | 5H |
| f | 2.38 (q) | 2.34 (q) | 2H |
| g | 0.92 (t) | 0.88 (t) | 3H |

IR (Nujol) $cm^{-1}$ 3000—2800 (s), 1610 (m), 1510 (m), 1460 (s), 1380 (s), 1250 (m), 1180 (m), 1040 (m), 820 (m), 770 (m), 705 (s), 670 (m).

(c) *Preparation of Tamoxifen from 1-[4-(2-chloroethoxy)phenyl]-1,2-diphenylbut-1-ene*

Z 1-[4-(2-chloroethoxy)phenyl]-1,2-diphenylbut-1-ene (100 mg) was heated with 30% w/v dimethylamine in ethanol (3 ml) for 3 days at 75° in a sealed vessel. The solvent and excess amine were removed by rotary evaporation. This product (97 mg, yield 94.7%) obtained from the reaction is already of

high purity but to prepare it in analytical purity, for NMR spectroscopy, it was chromatographed on a silica gel column chromatography, using as eluant chloroform/methanol 9:1 v/v.

Crystallisation from chloroform/hexane gave white needles (85 mg, 83.0% yield) identified by NMR as Z 1-[4-(2-dimethylaminoethoxy)phenyl]-1,2-diphenylbut-1-ene. Elemental analysis. Found C 84.2, H 8.1, N 3.5. $C_{26}H_{29}NO$ requires C 84.1, H 7.9, N 3.8%.

## Example 2

Preparation of Tamoxifen

(a) *Preparation of 4-(2-bromoethoxy)benzophenone*

Example 1, step (a) was repeated, using 1,2-dibromoethane instead of 1-bromo-2-chloroethane. 4-(2-Bromoethoxy)benzophenone was obtained as an off-white solid, m.p. 62—64°C, yield 70%.

(b) *Preparation of 1-[4-(2-bromoethoxy)phenyl]-1,2-diphenylbut-1-ene*

Example 1, step (b) was repeated, using 4-(2-bromoethoxy)benzophenone instead of the 4-(2-chloroethoxy)benzophenone, and with the minor variations that after addition of the petroleum ether, stirring was for 10 minutes and fractions having Rf = 0.45 were collected. The product obtained from the chromatography was a colourless syrup (0.45 g, 46%). The syrup was dissolved in isopropanol and white crystals formed on standing. These were shown to be a mixture of about 4:1 Z- and E 1-[4-(2-bromoethoxy)phenyl]-1,2-diphenylbut-1-enes, respectively.

Elemental analysis. Found C 71.0, H 5.5. $C_{24}H_{23}BrO$ requires C 70.8, H 5.7%. NMR, 60 mHz on EZ mixture:

| | E | Z |
|---|---|---|
| a and b | 3.37 (m) | 4.33 (m) |
| c or e | 7.05 (s) | 7.05 (s) 5H |
| d | 6.54—7.30 | 6.35—6.82 4H |
| e or c | 7.21 (s) | 7.21 (s) 5H |
| f | 2.40 (q) | 2.37 (q) 2H |
| g | 0.88 (t) | 0.83 (t) 3H |

IR (Nujol) $cm^{-1}$ 3000—2800 (s), 1610 (m), 1510 (m), 1460 (s), 1380 (s), 1290 (m), 1250 (s), 1175 (m), 820 (m), 770 (m), 715 (s).

(c) *Preparation of Tamoxifen from 1-[4-(2-bromoethoxy)phenyl]-1,2-diphenylbut-1-ene*

The preparation of Tamoxifen followed the procedure of Example 1, step (c) using the above-prepared bromoethoxy analogue in place of the chloroethoxy compound.

## Example 3

(a) *Preparation of 4-(2-chloroethoxy)benzophenone*

4-Hydroxybenzophenone (19.8 g), benzyl tri-n-butylammonium bromide (3.6 g) sodium hydroxide (8.0 g) water (75 ml) and 1,2-dichloroethane (75 ml) were vigorously stirred together at reflux for 18 hours. The mixture was cooled, the upper aqueous layer removed and the organic layer dried over magnesium sulphate. Evaporation of the solvent *in vacuo* afforded a red oil which crystallised from ethanol/water to yield 4-(2-chloroethoxy)benzophenone (22.8 g, 88% yield), m.p. 77—78°.

(b) *Preparation of 1-[4-(chloroethoxy)phenyl]-1,2-diphenylbut-1-ene*

Titanium tetrachloride (3.4 g, 18 mmol) was added dropwise to a stirred suspension of zinc powder (2.36 g, 36 mmol) in tetrahydrofuran (30 $cm^3$) at −10° under dry argon. The resulting dark mixture was heated to reflux, with stirring, and kept at that temperature for 1 hour. The solution was cooled to room temperature and a mixture of 4-(2-chloroethoxy)benzophenone (1.59 g, 6 mmol) and propiophenone (0.80 g, 6 mmol) dissolved in tetrahydrofuran (20 $cm^3$) was added. The mixture was refluxed with stirring for 2 hours.

After cooling to room temperature the dark mixture was poured into 10% potassium carbonate solution (200 $cm^3$), this was extracted with diethyl ether (3 × 100 $cm^3$), the organic extracts combined, dried and evaporated to a yellow oil.

The crude product was purified by column chromatography (silica gel, eluant hexane/ethyl acetate, 9:1), to give, as the major product, 1-[4-(2-chloroethoxy)phenyl]-1,2-diphenylbut-1-ene (1.46 g, 67%) as a colourless oil. This oil was dissolved in 2-propanol and deposited 1-[4-(2-chloroethoxy)phenyl]-1,2-diphenylbut-1-ene as the pure Z isomer, m.p. 64—65°, yielding (1.20 g, 54.2% overall yield) after filtration.

(c) *Preparation of Tamoxifen from 1-[4-(2-chloroethoxy)phenyl]-1,2-diphenylbut-1-ene*

Example 1, step (c) was repeated.

Reference Example

US Patent 2,914,562, Example 3, describes the preparation of 4-(2-N,N-dimethylaminoethoxy)benzophenone from 4-hydroxybenzophenone, using sodium methoxide and N,N-dimethylaminoethyl chloride. The product is reported to have b.p. 176—180°/0.3 mm. Hg but no yield is given. Chemical Abstract *53* 11296e mentions a 17% yield in a similar process. The above-cited Bristol-Myers Co. European Patent Application does not mention how 4-(2-N,N-dimethylaminoethoxy)benzophenone is prepared. The present inventors' best preparation of this compound is as follows.

The sodium salt of 4-hydroxybenzophenone (10 g), 1-N,N-dimethylamino-2-chloroethane (from its hydrochloride, 25.5 g) in toluene (100 ml) were heated under reflux for 24 hours. Filtration and then evaporation of the solvent yielded a brown oil, which was distilled *in vacuo* to yield 4-(2-N,N-dimethylaminoethoxy)benzophenone (9.7 g, 79.3% yield) b.p. 174—179/0.3 mm Hg %, yield 79.3%.

**Claims**

1. A process of preparing Tamoxifen of formula

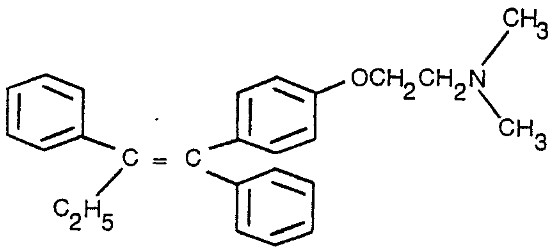

(1)

which comprises:

(1) reacting propiophenone with a 4-(2-haloethoxy)benzophenone of formula

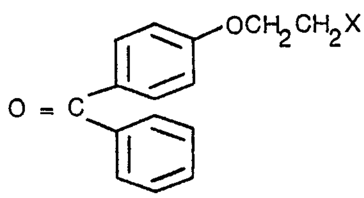

(2)

X being chlorine, bromine or iodine in a substantially dry and inert atmosphere and in a medium containing a reducible titanium compound and a reducing agent effective to generate titanium of substantially zero valence state to give the intermediate compound 1,2-diphenyl-1-[4-(2-haloethoxy)phenyl]-1-butene, of formula

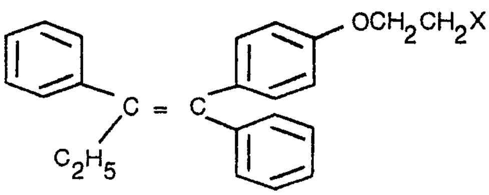

(3)

X being as defined above, and

(2) reacting the intermediate compound of formula (3) with dimethylamine.

2. A process according to Claim 1, wherein in step (1) the reducible titanium compound is titanium tetrachloride.

3. A process according to Claim 2, wherein the reducing agent is zinc.

4. A process according to Claim 1, wherein the step (1) the reducible titanium compound is titanium trichloride and the reducing agent is lithium.

5. A process according to claim 1, 2, 3 or 4, wherein step (1) is carried out in 1,2-dimethoxyethane or in tetrahydrofuran.

6. A process according to Claim 1, 2, 3, 4 or 5, wherein step (2) is carried out at a temperature of 60 to 100°C in a sealed vessel.

**Patentansprüche**

1. Verfahren zur Herstellung von Tamoxifen der Formel

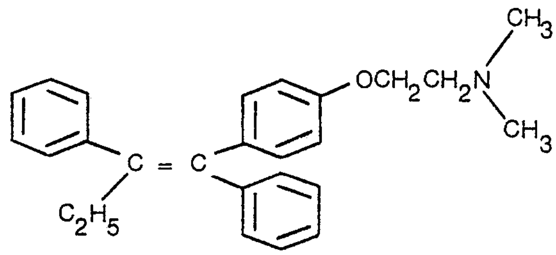

(I)

dadurch gekennzeichnet, daß man

(1) Propiophenon mit einem 4-(2-Halogenethoxy)-benzophenol der Formel

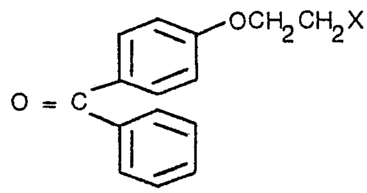

(2)

worin X Chlor, Brom oder Jod bedeutet, in einer praktisch trockenen und inerten Atmosphäre und in einem Medium umsetzt, das eine reduzierbare Titanverbindung und ein Reduktionsmittel enthält, das zur Bildung von Titan im Zustand von praktisch Nullwertigkeit wirksam ist, was die Zwischenverbindung 1,2-Diphenyl-1-[4-(2-halogenethoxy)-phenyl]-1-buten der Formel

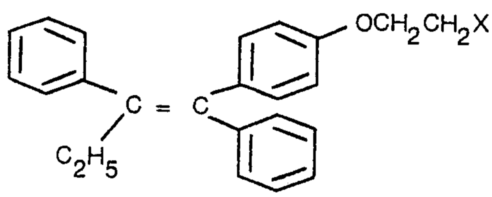

(3)

ergibt, worin X die obigen Bedeutungen hat und

(2) das Zwischenprodukt der Formel 3 mit Dimethylamin umsetzt.

2. Verfahren nach Anspruch 1, dadurch gekennzeichnet, daß in Stufe (1) die reduzierbare Titanverbindung Titantetrachlorid ist.

3. Verfahren nach Anspruch 2, dadurch gekennzeichnet, daß das Reduktionsmittel Zink ist.

4. Verfahren nach Anspruch 1, dadurch gekennzeichnet, daß in Stufe (1) die reduzierbare Titanverbindung Titantrichlorid und das Reduktionsmittel Lithium sind.

5. Verfahren nach Anspruch 1, 2 oder 4, dadurch gekennzeichnet, daß die Stufe 1 in 1,2-Dimethoxyethan oder in Tetrahydrofuran durchgeführt wird.

6. Verfahren nach Anspruch 1, 2, 3, 4 oder 5, dadurch gekennzeichnet, daß die Stufe (2) bei einer Temperatur von 60 bis 100°C in einem dicht verschlossenen Gefäß durchgeführt wird.

**Revendications**

1. Procédé de préparation de Tamoxifen, de formule

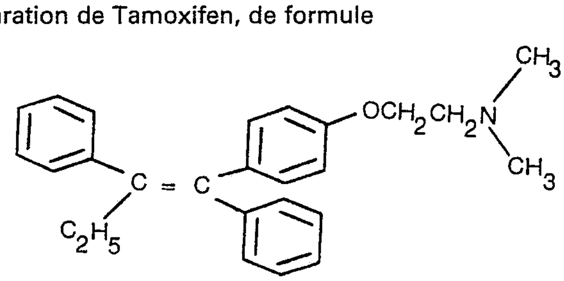

(I)

qui comprend

(1) la réaction de la propiophénone avec une 4-(2-halogénoéthoxy)benzophénone de formule

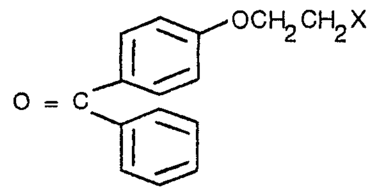

(2)

X représentant du chlore, du brome ou de l'iode, dans une atmosphère essentiellement sèche et inerte et dans un milieu contenant un composé de titane réductible et un agent réducteur capable d'engendrer efficacement du titane à un degré de valence sensiblement nul, pour obtenir le composé intermédiaire, le 1,2-diphényl-1[4-(2-halogénoéthoxy)phényl]-1-butène, de formule

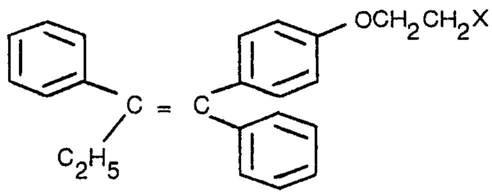

(3)

X étant tel que défini ci-dessus, et

(2) la réaction du composé intermédiaire de formule (3) avec la diméthylamine.

2. Procédé selon la revendication 1, dans lequel dans l'étape (1), le composé de titane réductible est du tétrachlorure de titane.

3. Procédé selon la revendication 2, dans lequel l'agent réducteur est du zinc.

4. Procédé selon la revendication 1, dans lequel dans l'étape (1), le composé de titane réductible est le trichlorure de titane, et l'agent réducteur est du lithium.

5. Procédé selon la revendication 1, 2, 3 ou 4, dans lequel on effectue l'étape (1) dans du 1,2-diméthoxyéthane ou dans du tétrahydrofuranne.

6. Procédé selon la revendication 1, 2, 3, 4 ou 5, dans lequel on effectue l'étape (2) à une température de 60 à 100°C dans un récipient fermé de manière étanche.